(19) Europäisches Patentamt
European Patent Office
Office européen des brevets

(11) Veröffentlichungsnummer: **0 114 575**
**B1**

(12) # EUROPÄISCHE PATENTSCHRIFT

(45) Veröffentlichungstag der Patentschrift: **03.02.88**

(51) Int. Cl.⁴: **C 07 D 239/48,** C 07 D 239/30, A 01 N 43/54

(21) Anmeldenummer: **83810608.6**

(22) Anmeldetag: **21.12.83**

(54) **2,4-Diamino-6-halogen-5-trifluormethylpyrimidine mit herbizider Wirkung.**

(30) Priorität: **28.12.82 CH 7588/82**

(43) Veröffentlichungstag der Anmeldung: **01.08.84 Patentblatt 84/31**

(45) Bekanntmachung des Hinweises auf die Patenterteilung: **03.02.88 Patentblatt 88/5**

(84) Benannte Vertragsstaaten: **BE CH DE FR GB IT LI NL**

(56) Entgegenhaltungen:
**EP-A-0 019 811**
**EP-A-0 023 033**
**DE-A-2 006 145**
**DE-A-2 630 140**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Tobler, Hans, Dr., Baselmattweg 157, CH- 4123 Allschwil (CH)**
Erfinder: **Hoegerle, Karl, Dr., Wasgenring 92, CH- 4055 Basel (CH)**

EP 0 114 575 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue 2,4-Diamino-6-halogen-5-trifluormethylpyrimidine mit herbizider und pflanzenwuchsregulierender Wirkung, die Herstellung dieser neuen Pyrimidine, ein Herbizides und den Pflanzenwuchs regulierendes Mittel welches sie als Wirkstoff enthält und deren Verwendung in Kulturpflanzenbeständen.

Pyrimidine mit herbizider oder sonst die Pflanzenphysiologie beeinflussender Wirkung sind in grosser Zahl bekannt geworden, als Beispiele seien genannt: J. prakt. Chemie 115, S. 292 (1927), DE-A-2 356 644 oder die EP-A-681 und EP-A-24 260. Halogenierte 4-Aminopyrimidine als Zwischenprodukte für Pharmazeutika sind ferner aus der EP-A-19 811, solche mit herbiziden Wirkungen aus den DE-A-2 006 145 und DE-A-2 630 140 bekannt geworden. In der EP-A-23 033 ist ein Weg zur Herstellung von 2,4,6-Trihalogenpyrimidin-5-carbonsäure beschrieben.

Die erfindungsgemässen Verbindungen hemmen in kleinen Aufwandmengen das vegetative Wachstum von Getreide, in grösseren Aufwandmengen wirken sie als Herbizide.

Die 2,4-Diamino-6-halogen-5-trifluormethylpyrimidine entsprechen der allgemeinen Formel (I)

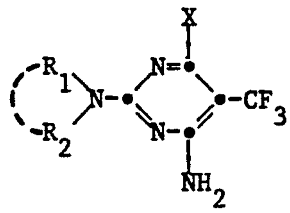

I

worin

X ein Halogenatom,

$R_1$ und $R_2$ unabhängig voneinander je Wasserstoff, $C_1$-$C_6$-Alkyl, $C_3$-$C_6$-Cycloalkyl oder zusammen eine $C_3$-$C_7$-Alkylenbrücke bedeuten.

Die Alkylgruppen umfassen sämtliche geradkettigen oder verzweigten Gruppen.

Die Pyrimidin-Verbindungen der Formel (I) weisen ausgesprochen selektivherbizide Eigenschaften im allgemeinen auf und erweisen sich als besonders vorteilhaft zum Bekämpfen von Unkräutern in Kulturen von Nutzpflanzen, insbesondere in Kulturen von Mais, Getreide aber auch von Soja, Baumwolle, Getreide wie Gerste, Hafer aber sowie auch von Zuckerrüben.

Bei genügend grosser Aufwandmenge ist jedoch auch totalherbizide Wirkung vorhanden. Die Anwendung kann sowohl im Vorauflauf - wie im Nachauflaufverfahren vorgenommen werden. Dabei können die Aufwandmengen in weiten Grenzen schwanken, z. B. zwischen 0,1 bis 10 kg Wirkstoff pro Hektar, vorzugsweise werden jedoch 0,5 bis 5 kg Wirkstoff pro Hektar eingesetzt.

Gute selektive Herbizidwirkung wurde teilweise aber schon bei Aufwandmengen von bloss 0,25 kg pro Hektar beobachtet.

Die Verbindungen der Formel (I) zeigen auch den Pflanzenwuchs regulierende Wirkung, wie beispielsweise Wuchshemmung in Getreide. Diese Wuchshemmung betrifft bloss das vegetative Wachstum. Man erhält so Getreide mit kürzeren dafür stärkeren Halmen, welche sich durch Wind und Unwetter nicht so leicht knikken oder umlegen lassen. Das generative Wachstum bleibt erhalten, ja man kann dank der besseren Klimaresistenz Ertragssteigerungen erzielen.

Bevorzugt sind diejenigen Pyrimidine der Formel I, in denen X Chlor oder Fluor, insbesondere Fluor bedeutet und $R_1$ und $R_2$ die gegebene Bedeutung haben; speziell aufgefallen sind folgende Verbindungen:

2,4-Diamino-6-fluor-5-trifluormethylpyrimidin,

4-Amino-6-fluor-2-methylamino-5-trifluormethylpyrimidin und

4-Amino-2-dimethylamino-6-fluor-5-trifluormethylpyrimidin.

Die Herstellung der 2,4-Diamino-6-halogen-5-trifluorpyrimidine geschieht in seinen letzten Phasen in an sich bekannter Weise.

Ein 2,4,6-Trihalogen-5-trifluormethylpyrimidin der Formel (II)

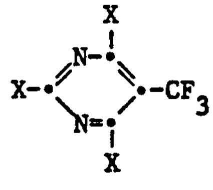

II

worin X ein Halogenatom, vorzugsweise Chlor oder Fluor verkörpert, wird in einem den Reaktionsteilnehmern gegenüber inerten Lösungsmittel bei einer Temperatur zwischen -10° und +50°C mit 2-3 Mol einer wässrigen Lösung eines Amins der Formel (III) behandelt

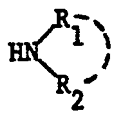

III

worin

$R_1$ und $R_2$ die oben gegebene Bedeutung haben und das Endprodukt durch abtrennen der wässrigen Phase und einengen des Lösungsmittels isoliert. Dabei entsteht zur Hauptsache 2-Amino-4,6-dihalogen-5-trifluormethylpyrimidin der Formel (IV)

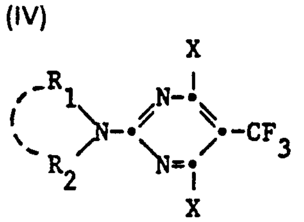

IV

worin $R_1$, $R_2$ und X die oben gegebene Bedeutung haben, neben kleineren Anteilen von 4-Amino-2,6-dihalogen-5-trifluormethylpyrimidin und 2,4-Diamino-6-halogen-5-trifluormethylpyrimidin, welche sich durch Chromatographie gut trennen lassen. Das 2-Amino-4,6-dihalogen-5-trifluormethylpyrimidin der Formel (IV) wird dann in einem den Reaktionsteilnehmern gegenüber inerten Lösungsmittel bei einer Temperatur zwischen +20 und +100°C mit 2-3 Mol wässriger Ammoniaklösung behandelt wobei nach Abtrennung der wässrigen Phase als Hauptprodukt das 2,4-Diamino-6-halogen-5-trifluormethylpyrimidin der Formel (I) anfällt, zusammen mit geringen Mengen von 4,6-Diamino-2-halogen-5-trifluormethylpyrimidin und stereoisomerem 2,4-Diamino-6-halogen-5-trifluormethylpyrimidin, worin die Aminogruppen die Stellung vertauscht haben, als Nebenprodukte, welche sich durch Chromatographie leicht trennen lassen.

Das Verfahren zur Herstellung der neuen 2,4-Diamino-6-halogen-5-trifluormethylpyrimidin der Formel (I) ist demnach dadurch gekennzeichnet, dass man ein 2,4,6-Trihalogen-5-trifluormethylpyrimidin der Formel (II), in einem inerten Lösungsmittel bei einer Temperatur zwischen -10° und +50°C mit 2-3 Mol Äquivalenten einer wässrigen Lösung eines Amins der Formel (III) behandelt, das entstandene 2-Amino-4,6-dihalogen-5-trifluormethylpyrimidin isoliert und in einem inerten Lösungsmittel bei einer Temperatur von +20° bis +100°C mit 2-3 Mol Äquivalenten wässriger Ammoniaklösung behandelt und anschliessend das Endprodukt isoliert.

Als inerte Lösungsmittel kommen vorteilhaftersweise Kohlenwasserstoffe, Halogen Kohlenwasserstoffe oder Äther in Frage, wie beispielsweise Toluol, Benzol, Chloroform, Methylenchlorid Äthylenchlorid, Äther, Dioxan, Tetrahydrofuran oder Mischungen derselben miteinander. Es ist von Vorteil, wenn das Lösungsmittel mit Wasser nicht mischbar ist weil dann das Zwischen- oder Endprodukt durch Verwerfen der wässrigen Phase und Konzentrieren der organischen Phase leicht zu isolieren ist.

Die für diese Herstellung benötigten Ausgangs- und Zwischenprodukte und ihre Herstellung sind grösstenteils neu.

Zur Herstellung der 2,4,6-Trihalogen-5-trifluormethyl-pyrimidine der Formel (II) geht man z. B. von den aus der DE-A-2 310 334 bekannten 2,4,6-Trihalogen-5-formyl-pyrimidinen oder einem ähnlichen Trihalogen-pyrimidin der Formel (V) aus

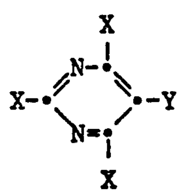

V

worin X ein Halogenatom, vorzugsweise Chlor und Y Methyl, Formyl oder Halogenmethyl ist, oxidiert dies bei Temperaturen zwischen 10° und 100°C, bevorzugt bei 20° und 50°C, in Gegenwart von Chromtrioxid oder einem Salz, das unter den Reaktionsbedingungen Chromtrioxid bildet und in Gegenwart von rauchender oder zumindest 98 %-iger Schwefelsäure zur 2,4,6-Trihalogen-pyrimidin-5-carbonsäure der Formel (VI)

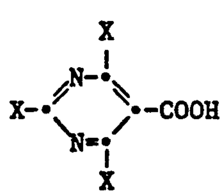

VI

worin X ein Halogenatom bedeutet und unterwirft diese Verbindung gegebenenfalls einer Umhalogenierung.

Nach diesem Verfahren lassen sich die Verbindungen der Formel (VI), vor allem die entsprechenden Chlorderivate, auf einfache und wirtschaftliche Weise in guten bis sehr guten Ausbeuten herstellen. 2,4-Dihalogenpyrimidin-5-carbonsäurehalogenide konnten bisher nur auf sehr umständliche Weise aus Cyanacetylharnstoff mit dem Bredereck-Komplex aus Dimethylsulfat und Dimethylformamid über 5-Cyanouracil und die Uracil-5-carbonsäure hergestellt werden [vgl. z. B. britische Patentschriften 1.123.762 und 1.182.086]. 2,4,6-Trichlorpyrimidin-5-carbonsäurechlorid ist in der DE-B-21 32 963 namentlich als Reaktivkomponente erwähnt; es wird jedoch nicht angegeben, wie diese Verbindungen hergestellt werden könnte.

Als unter den Reaktionsbedingungen Chromtrioxid abgebende Salze können zum Beispiel Kaliumchromat, Kalium- oder Natriumdichromat eingesetzt werden. Bevorzugt wird die Umsetzung in Gegenwart von Chromtrioxid durchgeführt.

Als rauchende Schwefelsäure verwendet man vorzugsweise eine solche mit einem $SO_3$-Gehalt von 20-80 %. Besonders bevorzugt ist rauchende Schwefelsäure mit einem $SO_3$-Gehalt von 25 %. Die Schwefelsäure dient dabei als Lösungsmittel; sie kann jedoch auch mit einem unter den Reaktionsbedingungen inerten und nicht oxidierenden Lösungsmittel, wie Tetrachlorkohlenstoff oder Tetrachloräthan verdünnt werden.

Die Ausgangsprodukte der Formel (V) sind bekannt oder können auf an sich bekannte Weise hergestellt werden.

Bevorzugt verwendet man als Verbindung der Formel (V) 5-Formyl-2,4,6-trichlorpyrimidin, 5-Methyl-2,4,6-trichlorpyrimidin oder 5-Chlormethyl-2,4,6-trichlorpyrimidin.

Verbindungen der Formel (VIa), worin X ein Halogen, vorzugsweise Chlor oder Brom bedeutet R, für Wasserstoff und Y für Carboxyl oder Chlorcarbonyl stehen,

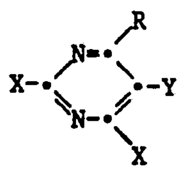

VIa

können ebenfalls in Verbindungen der Formel (VI) bzw. in die entsprechenden Säurehalogenide überführt werden, indem man die erstgenannten Verbindungen bei einer Temperatur von 20° bis 350°C in Gegenwart von Aktivkohle und eines Friedel-Crafts-Katalysators mit einem Chlor, Fluor oder Brom einführenden Mittel behandelt. So erhaltene Säuren der Formel (VI) oder deren Säurehalogenide, worin mindestens eines der X Chlor bedeutet, können gewünschtenfalls auch einer Umhalogenierung unterworfen werden. Bevorzugt ist die Nachchlorierung der Verbindung der Formel (VIa), in der R Wasserstoff, X Chlor und Y Chlorcarbonyl bedeuten.

Geeignete Friedel-Crafts-Katalysatoren für die obige Umsetzung sind z. B. Eisen(II)chlorid, Eisen(III)chlorid, Antimon(V)chlorid und Zinn(II)chlorid. Bevorzugt verwendet man als Katalysator Eisen(III)-chlorid im Gemisch mit Aktivkohle. Dabei liegen die Reaktionstemperaturen vorzugsweise zwischen 150 und 160°C. Der Katalysator wird im allgemeinen in einer Menge von 1 bis 10 Mol.%, bezogen auf die definitionsgemässe Ausgangsverbindung der Formel I, verwendet.

Als Chlor, Fluor und/oder Brom einführende Mittel kommen z. B. $Cl_2$, $Br_2$, $F_2$, Phosphor(V)chlorid, -bromid und -fluorid oder Sulfurylchlorid in Betracht. Bevorzugt verwendet man ein Chlor einführendes Mittel, vor allem $Cl_2$.

Verbindungen der Formel (VI), worin X Chlor darstellt, können gewünschtenfalls bis zum Ersatz von einem oder mehreren Chloratomen mit einem Bromierungs- oder Fluorierungsmittel, wie Phosphortribromid, wasserfreiem Fluorwasserstoff, Alkalimetallfluoriden, Silberdifluorid, Antimon(III)fluorid, Antimon(V)-fluorid oder Kaliumfluorosulfinat, umgesetzt werden. So können z. B. die Verbindungen der Formel (VI), aber auch (VIa), worin die X Chlor bedeutet, in die Brom- oder Fluoranalogen übergeführt werden, indem man die genannten Verbindungen der Formel (VI) durch Umsetzung mit Phosphortribromid, das auch als Lösungsmittel dienen kann, in das 2,4-Dibrom- oder 2,4,6-Tri-brompyrimidin-5-carbonsäurebromid überführt oder indem man solche Verbindungen durch Umsetzung mit einem der oben genannten Fluorierungsmittel, gegebenenfalls in Gegenwart eines hochsiedenden aprotischen organischen Lösungsmittels in das 2,4-Difluor- oder 2,4,6-Trifluorpyrimidin-5-carbonsäurefluorid überführt. Geeignete Lösungsmittel für diese Umhalogenierungsreaktionen sind z. B. aromatische Kohlenwasserstoffe, wie Toluol und Xylole; N,N-Dialkylamide von aliphatischen Monocarbonsäuren mit 1-3 C-Atomen im Säureteil, wie N,N-Dimethylformamid, N,N-Dimethylacetamid und N,N-Diäthylacetamid; cyclische Aether und cyclische Amide, wie Tetrahydrofuran, Tetrahydropyran, N-Methyl-2-pyrrolidon und N-Acetyl-2-pyrrolidon; Hexamethylphosphorsäuretriamid (Hexametapol); N,N,N',N'-Tetramethylharnstoff, Tetrahydrothiophendioxid (Sulfolan). Die Reaktionstemperaturen für die Umhalogenierung liegen zweckmässig zwischen 20 und 250°C, bevorzugt zwischen 50 und 150°C.

Um die 2,4,6-Trihalogen-5-trifluormethylpyrimidin Ausgangsstoffe der Formel (II) zu erhalten, muss die 2,4,6-Trihalogenpyrimidin-5-carbonsäure der Formel (VI) oder ein Säurehalogenid davon im Autoklaven bei einer Temperatur von 50-200°C in Gegenwart von 2-10 Mol Äquivalenten Schwefeltetrafluorid und von 3,4-Mol Äquivalenten Fluorwasserstoffsäure als Verdünnungsmittel während 5-15 Stunden behandelt wird. Solche

Fluorierungen der Carboxylgruppe oder allenfalls eines Carbonylhalogenides sind aus der Literatur bekannt, siehe Org. Reactions 21 Iff; Angewandte Chemie 74, 742 (1962) oder J. Am. Chem.Soc. 82, 543 (1960). Ebenso ist das dabei vornehmlich entstehende 2,4,6-Trifluor-5-trifluormethylpyrimidin aus J. Chemie. Soc.(C) 1970, 1280, bekannt.

Die Erfindung betrifft auch herbizide und pflanzenwachstumsregulierende Mittel welche einen neuen Wirkstoff der Formel (I) enthalten, sowie Verfahren zur pre- und post-emergenten Hemmung des Pflanzenwuchses von monokotylen und dikotylen Pflanzen, insbesondere Gräsern, tropischen Bodenbedeckern und Tabakgeiztrieben.

Die Verbindungen der Formel (I) werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel (I) und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Äther und Ester wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe z. B. für Stäubemittel und dispergierbare Pulver, werden in der Regel natürliche Gesteinsmehle verwendet, wie Calcit, Talkum, Kaolin, Montmorillonit oder Attapulgit. Zu Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtiogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können wowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Öl- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fetthalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-äthylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5 Äthylenglykoleinheiten.

Als Beispiele nichtionischer Tenside seien Nonylphenolpolyäthoxyäthanole, Ricinusölpolyglykoläther, Polypropylenpolyäthylenoxidaddukte, Tributylphenoxypolyoxyäthanol, Polyäthylenglykol und

Octylphenoxypolyäthoxyäthanol erwähnt.

Ferner kommen auch Fettsäureester von Polyoxyäthylensorbitan wie das Polyoxyäthylensorbitan-trioleat oder Phospholipide in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige gegebenenfalls halogenierte Alkyl-, Benzyl oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi-(2-chloräthyl)-äthylammoniumbromid.

Die in der Formulierungstechnik gebräuchlichen Tenside sind u.a. in folgenden Publikationen beschrieben:
"Mc Cutcheon's Detergents and Emulsifiers Annual" MC Publishing Corp., Ringwood, New Jersey, 1979;
M. and J. Ash, "Encyclopedia of Surfacetants" Vol. I-VI, Chemical Publishing Co. Inc., New York, 1980-81.
H. Stache "Tensid Taschenbuch" 2. Auflage, C. Hanser Verlag München und Wien 1981.

Die Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

**Bereitung der Zwischenprodukt:**

a) Verfahren zur Herstellung von 2,4,6-Trifluor-5-trifluor-methylpyrimidin

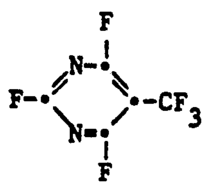

Ein 0,3 l Monel-Autoklav wird mit 30,5 g 2,4,6-Trichlorpyrimidin-5-carbonsäure (0,134 Mol), 144 g Schwefeltetrafluorid, $SF_4$ (1,3 Mol) und 14,4 g Fluorwasserstoffsäure, HF (0,72 Mol) beladen und dann 7 Stunden auf 110°C und 12 Stunden auf 160°C erhitzt. Nach dem Abkühlen bläst man das Reagens langsam aus, wobei gekühlt wird. Der Rückstand wird in Dichlormethan aufgenommen. Diese Lösung wird mit gesättigter Natrium-Bicarbonat-Lösung neutralisiert. Die Mischung wird dann über Hyflo filtriert und die wässrige Phase abgetrennt und verworfen. Die organische Phase wird getrocknet und auf 50 ml Volumen eingeengt. Der Rückstand wird schliesslich einer fraktionierten Destillation unterworfen. Man isoliert schliesslich 5,3 g einer fast farblosen Flüssigkeit von hoher Dichte und Siedepunkt 78°C (19,5 % der Theorie). Der Lösungsmittelvorlauf, welcher bei 37-39°C siedet, enthält ebenfalls Anteile an 2,4,6-Trifluor-5-trifluormethylpyrimidin.

| | | | | | |
|---|---|---|---|---|---|
| Analyse: | $C_5F_6N_2$ | M.G. 202,96 | | | |
| Ber.: | C 29,72 % | - | | F 56,41 % | N 13,87 % |
| Gef.: | C 29,07 % | Cl 1,57 % | | F 55,70 % | N 13,39 % |

NMR Spektrum: $^{19}F$-NMR ($CDCl_3$, Hexafluorbenzol als interner Standard, Chemische Verschiebungen auf $CCl_3F$ als interner Standard ungerechnet $^{14}N$-entkoppelt):
- 58,3 ppm ($tJ_{F_3C,F-C(4,6)} = 17,5$ Hz, ferner $J_{F_3C-F-C(2)} = 1$ Hz:$CH_3$);
- 49,1 ppm ($qJ_{F-C(4,6)-F_3C} = 17,5$ Hz: F-C(4,6));
- 34,5 ppm (s: F-C(2))
siehe dazu auch J. Chem. Soc. (C) 1970, 1280.

b) Herstellung von 2-Amino-4,6-difluor-5-trifluormethylpyrimidin und 4-Amino2,6-difluor-5-trifluormethylpyrimidin

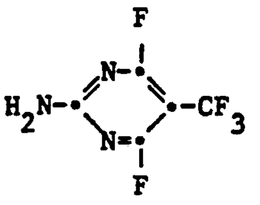
und
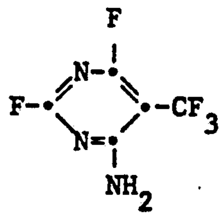

Zu einer Lösung bestehend aus 2,4,6-Trifluor-5-trifluormethylpyrimidin (welche gemäss Beispiel 1 aus 22,7 g 2,4,6-Trichlorpyrimidin-5-carbonsäure erhalten wurden) in 100 ml Methylenchlorid gibt man unter kräftigem Rühren bei Raumtemperatur 30 ml 25 %-ige wässrige Ammoniaklösung, was eine lebhafte exotherme Reaktion auslöste. Nach 10 Minuten weiterrühren wird die wässrige Phase abgetrennt, die organische Phase mit Wasser gewaschen, über Natriumsulfat getrocknet und eingedampft. Der Rückstand kristallisiert (16 g). Man löst ihn

und chromatographiert ihn mit Äther/Hexan 1 : 1 über eine 300 g Kieselgel-Säule. Man isoliert schliesslich 3,8 g der isomeren Amino-difluor-5-trifluorpyrimidinmischung welche die Einzelkomponenten im Verhältnis 7 : 3 enthält (gaschromatographisch ermittelt) und bei 125-155°C schmilzt.

Ausbeute: 44 % bezogen auf die 2,4,6-Trichlorpyrimidincarbonsäure, nebst einer nicht bestimmten Menge 2,4-Diamino-6-fluor-5-trifluor-methylpyrimidin.

| | | | | | |
|---|---|---|---|---|---|
| Analyse: | Ber. | C 30,16 | H 1,01 | N 21,11 | F 47,72 |
| | Gef. | C 30,18 | H 1,02 | N 21,08 | F 46,78. |

c) Herstellung von 2,4,6-Trichlorpyrimidin-5-carbonsäurre

Zu einer Lösung von 63,3 g (0,3 Mol) 2,4,6-Trichlor-5-formylpyrimidin in 300 ml rauchender Schwefelsäure ($SO_3$ -Gehalt 25 %) werden 24 g (0,24 Mol) Chromtrioxid in Schuppen gegeben. Die Mischung wird während 20 Stunden gerührt. Die Temperatur steigt innerhalb ca. einer Stunde auf 45-50°C, um dann wieder auf Raumtemperatur (20-25°C) abzufallen. Die Reaktionslösung wird auf Eiswasser gegossen, der Niederschlag wird abfiltriert und mit wenig Eiswasser gewaschen. Das Reaktionsprodukt wird in Diäthyläther gelöst, über Natriumsulfat getrocknet, und die Lösung wird zur Trockene eingedampft. Man erhält 55,4 g (81,2 %) 2,4,6-Trichlorpyrimidin-5-carbonsäure vom Smp. ca. 150°C. Durch Umkristallisieren aus Chloroform kann der Smp. auf 155-157°C angehoben werden.

Analyse für $C_5HCl_3N_2O_2$ (Molgewicht 227,43):

| | | | | | |
|---|---|---|---|---|---|
| berechnet | C 26,41 % | H 9,45 % | N 12,32 % | O 14,07 % | Cl 46,77 % |
| gefunden | C 26,28 % | H 9,48 % | N 12,23 % | O 14,69 % | Cl 45,93 %. |

$^{13}C$-NMR-Spektrum ($CD_3CN$, TMS interner Standard):
163,04 ppm (COOH), 160,2 ppm (C(4)) und (C(6)), 159,9 ppm (C(2)), 127,8 ppm (C(5)).

Das in obigen Beispiel verwendete Ausgangsprodukt kann nach dem in der DE-A-2 310 334 beschriebenen Verfahren hergestellt werden.

2,4,6-Trichlorpyrimidin-5-carbonsäure kann nach dem oben beschriebenen Verfahren auch unter folgenden Reaktionsbedingungen erhalten werden:

| Oxidations-mittel | Reaktionsmedium | Ausbeute an 2,4,6-Trichlor-pyrimidin-5-carbonsäure |
|---|---|---|
| $CrO_3$ | 98 %-ige $H_2SO_4$ | 50 % d.Th. |
| $CrO_3$ | rauchende $H_2SO_4$ ($SO_3$-Gehalt 65 %) | 60 % d.Th. |
| $K_2Cr_2O_7$ | rauchende $H_2SO_4$ ($SO_3$-Gehalt 25 %) | 20 % d.Th. |

d) Herstellung von 2,4,6-Trichlorpyrimidin-5-carbonsäure

19,8 g (0,1 Mol) 5-Methyl-2,4,6-trichlorpyrimidin werden in 100 ml rauchender Schwefelsäure ($SO_3$-Gehalt 25 %) eingetragen. Unter Erwärmung auf 38°C geht der Festkörper in Lösung. Dann werden 15,8 g (0,15 Mol) 95 %-iges Chromtrioxyd in Schuppen zugegeben und das Reaktionsgemisch durch zeitweises schwaches Kühlen auf 45-50°C gehalten. Nach 2 Stunden ist die exotherme Reaktion abgeklungen. Die Suspension wird noch 15 Stunden bei 45-50°C ausgerührt und dann auf 200 g Eis getropft. Die Temperatur wird durch zusätzliche Aussenkühlung auf maximal 5°C gehalten. Der entstandene Niederschlag wird filtriert, trocken gesaugt und in Diäthyläther gelöst. Die Ätherlösung wird über Magnesiumsulfat getrocknet und zur Trockene eingeengt. Man erhält 5,2 g (23 % d.Th.) 2,4,6-Trichlorpyrimidin-5-carbonsäure vom Smp. 155-157°C. Das im obigen Beispiel verwendete Ausgangspyrimidin kann gemäss Chem. Ber. 90, 728 (1957) hergestellt werden.

**Beispiel 1:**

Herstellung von 2,4-Diamino-6-fluor-5-trifluormethylpyrimidin

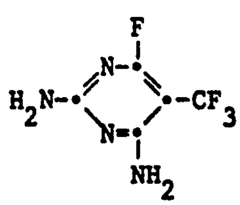

Die nach dem Verfahren b) erhaltene Mischung von 5,3 g 2-Amino-4,6-difluor-5-trifluormethylpyrimidin und 4-Amino-2,6-difluor-5-trifluormethyl-pyrimidin (Verhältnis 7 : 3, keine Zuordnung) wird in 100 ml Tetrahydrofuran gelöst und unter Rühren bei Raumtemperatur mit 10 ml 25 %-iger wässriger Ammoniaklösung versetzt. Die Temperatur sinkt erst, steigt dann auf 30°C an. Nach 30 Minuten Ausrühren gibt man Äther zum Reaktionsgemisch, trennt die organische Phase ab und wäscht 3x mit Wasser. Dann wird über Natriumsulfat getrocknet und eingedampft. Der Rückstand wird mit Äther/Hexan 1 : 1 über eine 300 g Kieselgel-Säule chromatographiert. Man isoliert 4,75 g 2,4-Diamino-6-fluor-5-trifluormethylpyrimidin (91 % der Theorie), welches bei 170-174°C schmilzt.

Analyse: $C_5H_4F_4N_4$ (M.G. 196,11)

| | | | | |
|---|---|---|---|---|
| Ber.: | C 30,62 % | H 2,06 % | N 28,57 % | F 38,75 % |
| Gef.: | C 31,01 % | H 2,04 % | N 28,44 % | F 38,05 %. |

Es wird ferner 0,3 g (5 % der Theorie) eines bei 220-225°C sublimierenden Nebenproduktes isoliert welches auf Grund des Massenspektrums und der Analyse als 4,6-Diamino-2-fluor-5-trifluormethylpyrimidin identifiziert wurde.

**Beispiel 2:**

Herstellung von 4-Amino-2-methylamino-6-fluor-5-trifluormethylpyrimidin

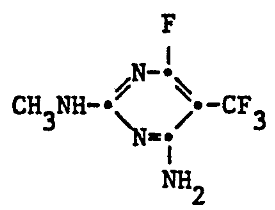

Eine Lösung von 2,4,6-Trifluor-5-trifluormethylpyrimidin (hergestellt gemäss Verfahren a) aus 46 g 2,4,6-Trichlorpyrimidin-5-carbonsäure) in 200 ml Methylenchlorid wird unter starkem Rühren bei Raumtemperatur mit 35 ml 40 %-iger wässriger Methylamin-Lösung versetzt. Nach dem alles zugegeben ist, lässt man 10 Minuten nachrühren und trennt dann die wässrige Phase ab. Die organische Phase wird eingeengt, der Rückstand in 150 ml Tetrahydrofuran aufgenommen und unter Rühren bei Raumtemperatur mit 40 ml einer wässerigen 40 %-igen Ammoniak-Lösung versetzt. Nach dem Abklingen der exothermen Reaktion, nach ca. 75 Minuten, gibt man Äther zum Reaktionsgemisch, trennt die wässrige Phase ab, wäscht die organische Phase mit Wasser, trocknet und dampft sie ein. Der Rückstand wird durch Chromatographie über eine 2 kg Kieselgel-Säule mit Äther/Hexan 1 : 1 gereinigt. Man trennt so in 3 Verbindungen, die einzeln isoliert werden.

Die Hauptfraktion besteht aus 13,5 g 4-Amino-2-methylamino-6-fluor-5-trifluormethylpyrimidin (32 % der Theorie, bezogen auf 2,4,6-Trichlorpyrimidin-5-carbonsäure) welche als Kristalle vom Schmelzpunkt 160-162°C anfallen.

$^{13}$C-NMR (DMSO-$Cl_6$) u.a. $\delta_{CH_3}$ 28,0 ppm

Analyse: $C_6H_6F_4N_4$ (M.G. 210,13)

| | | | | |
|---|---|---|---|---|
| Ber.: | C 34,30 % | H 2,88 % | N 26,67 % | F 36,17 % |
| Gef.: | C 34,29 % | H 2,83 % | N 26,52 % | F 35,97 %. |

Eine zweite Fraktion besteht aus 3,2 g 2-Amino-4-methylamino-6-fluor-5-trifluormethylpyrimidin (7,5 % der Theorie, bezogen auf 2,4,6-Trichlorpyrimidin-5-carbonsäure), welches bei 100-105°C schmilzt und durch das NMR-Spektrum und der Analyse identifiziert wird.

Als dritte Fraktion fällt 2,3 g 2,4-Bis-Methylamino-6-fluor-5-trifluormethylpyrimidin an (5 % der Theorie, bezogen auf 2,4,6-Trichlorpyrimidin-5-carbonsäure) welches bei 142-145°C schmilzt und durch das NMR-Spektrum und die Analyse identifiziert wird.

In analoger Weise zu dem Beispielen 1 und 2 werden folgende Verbindungen hergestellt:

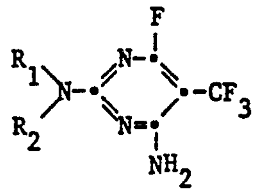

| No. | $R_1$ | $R_2$ | |
|---|---|---|---|
| 1 | H | H | Smp. 170-174°C Beisp. 3 |
| 2 | $CH_3$ | H | Smp. 160-162°C Beisp. 4 |
| 3 | $CH_3$ | $CH_3$ | Smp. 145°C |
| 4 | $C_2H_5$ | H | |
| 5 | $C_3H_7n$ | H | |
| 6 | Cycloproyl | H | |
| 7 | $C_2H_5$ | $C_2H_5$ | |
| 8 | $C_4H_9n$ | H | |
| 9 | $C_4H_9iso$ | H | |
| 10 | $C_4H_9t$ | H | |
| 11 | $C_6H_{13}n$ | H | |
| 12 | Cyclopentyl | H | |
| 13 | Cyclohexyl | H | |
| 14 | $C_3H_7n$ | $C_3H_7n$ | |
| 15 | $C_3H_7iso$ | H | |
| 16 | $C_4H_9sec$ | H | |
| 17 | Cyclobutyl | H | |
| 18 | $-C_5H_{10}-$ | | |
| 19 | $-C_4H_8-$ | | |
| 20 | $-C_3H_6-$ | | |

**Beispiel 3:**

Formulierungsbeispiele

Die Verbindungen der Formel (I) werden im allgemeinen nicht als solche in der Landwirtschaft eingesetzt. Man verwendete gebrauchsfertige formulierte Mittel, welche entweder direkt oder mit Wasser verdünnt eingesetzt werden können.

**Formulierungsbeispiele für flüssige Wirkstoffe der Formel (I) (% = Gewichtsprozent):**

| **1. Emulsions-Konzentrate** | a) | b) | c) |
|---|---|---|---|
| Wirkstoff | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

**2. Lösungen**

| | a) | b) | c) | d) |
|---|---|---|---|---|
| Wirkstoff | 80 % | 10 % | 5 % | 95 % |
| Aethylenglykolmonomethyläther | 20 % | - | - | - |
| Polyäthylenglykol M G 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190° C) | - | - | 94 % | - |

Die Lösungen sind zur Anwendung in Form kleinster Tropfen geeignet.

**3. Granulate**

| | a) | b) |
|---|---|---|
| Wirkstoff | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

**4. Stäubemittel**

| | a) | b) |
|---|---|---|
| Wirkstoff | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

**Formulierungsbeispiele für feste Wirkstoffe der Formel (I) (% = Gewichtsprozent):**

**5. Spritzpulver**

| | a) | b) | c) |
|---|---|---|---|
| Wirkstoff | 20 % | 50 % | 75 % |
| Na-Ligninsulfonat | 5 % | 5 % | - |
| Na-Laurylsulfat | 3 % | - | 5 % |
| Na-Diisobutylnaphtalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

**6. Emulsions-Konzentrate**

| | |
|---|---|
| Wirkstoff | 10 % |
| Octylphenolpolyäthylenglykoläther (4-5 Mol AeO) | 3 % |
| Ca-Dodecylbenzolsulfonat | 3 % |
| Ricinusölpolyäthylenglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

**7. Stäubemittel**

| | a) | b) |
|---|---|---|
| Wirkstoff | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

**8. Extruder-Granulat**

| | |
|---|---|
| Wirkstoff | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

**9. Umhüllungs-Granulat**

| | |
|---|---|
| Wirkstoff | 3 % |
| Polyäthylenglykol (M G 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

**10. Suspensions-Konzentrat**

| | |
|---|---|
| Wirkstoff | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %-ige wässrige Formaldehyd-Lösung | 0,2 % |
| Silikonöl in Form einer 75 %-igen wässrigen Emulsion | 0,8 % |
| Wasser | 32 % |

Der feingemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Beispiel 4:**

**Nachweis der herbiziden Wirkung**

(a) Vorauflaufversuch (pre-emergente Wirkung)

Im Gewächshaus werden die Samen von Avena sativa, Sinapis alba, Setaria italica und Stellaria media in Töpfe von ca. 11 cm Durchmesser gesät. Kurz darauf wird die Erdoberfläche mit einer wässrigen Emulsion der Wirkstoffe, erhalten aus einem 25 %-igen Emulsionskonzentrat behandelt. Es wird eine Aufwandmenge, die 4 kg Wirksubstanz pro Hektar entspricht, versprüht. Die Töpfe werden im Gewächshaus bei einer Temperatur von 22-25°C und 50-70 % relativer Luftfeuchtigkeit gehalten. Der Versuch wird nach 3 Wochen ausgewertet und die Resultate gemäss folgender Notenskala bonitiert:

| | |
|---|---|
| 1 | = Pflanzen nicht gekeimt oder total abgestorben |
| 2-3 | = sehr starke Wirkung |
| 4-6 | = mittlere Wirkung |
| 7-8 | = geringe Wirkung |
| 9 | = keine Wirkung (wie unbehandelte Kontrolle) |

Die Resultate sind wie folgt:

| Verbindung No. | 1 | 2 | 3 |
|---|---|---|---|
| Pflanze: | | | |
| Avena sativa | 1 | 1 | 8 |
| Sinapis alba | 1 | 1 | 2 |
| Setaria italica | 1 | 1 | 3 |
| Stellaria media | 1 | 1 | 2 |

b) Nachauflaufversuch (post-emergente Wirkung)

Im Gewächshaus werden in Töpfen von 11 cm Durchmesser die Pflanzen Avena sativa, Setaria italica, Lolium perenne, Solanum lycopersicum, Sinapis alba, Stellaria media und Phaseolus vulgaris gezogen, bis sie das 4-6 Blatt-Stadium erreicht haben, was nach ca. 2 Wochen der Fall ist. Dann werden sie mit einer wässrigen

Wirkstoffemulsion in einen Dosierung von 4 kg Wirksubstanz pro Hektar gespritzt und dann bei 24°-26°C und 45-60 % relativer Luftfeuchtigkeit gehalten. 15 Tage nach der Behandlung wird der Versuch ausgewertet und das Ergebnis wie im Vorauflauf-Versuch nach derselben Notenskala ausgewertet. Die Resultate sind wie folgt:

| Verbindung | No. | 1 | 2 | 3 |
|---|---|---|---|---|
| Pflanze: | | | | |
| Avena sativa | | 1 | 1 | 9 |
| Setaria italica | | 1 | 1 | 5 |
| Lolium perenne | | 2 | 2 | 7 |
| Solanum lycopersicum | | 1 | 1 | 1 |
| Sinapis alba | | 1 | 1 | 2 |
| Stellaria media | | 1 | 1 | 2 |
| Phaseolus vulgaris | | 5 | 3 | 7 |

**Wuchshemmung bei Gräsern**

In Kunststoffschalen mit Erde-Torf-Sand-Gemisch (6 : 3 : 1) wurden Samen der Gräser Lolium perenne, Poa pratensis, Festuca ovina und Dactylis glomerata ausgesät und normal bewässert. Die aufgelaufenen Gräser wurden wöchentlich bis auf 4 cm Höhe zurückgeschnitten und 40 Tage nach der Aussat und 1 Tag nach dem letzten Schnitt mit wässrigen Spritzbrühen eines Wirkstoffes der Formel I bespritzt. Die Wirkstoffmenge betrug umgerechnet 5 kg Aktivsubstanz pro Hektar. 10 und 21 Tage nach der Applikation wurde das Wachstum der Gräser beurteilt. Die Verbindungen 1, 2 und 3 hemmen den Graswuchs zwischen 13 und 22 %.

**Wuchshemmung bei Getreide**

In Kunststoffbechern wurde Sommerweize (Triticum aestivum), Sommergerste (Hordeum vulgare) und Roggen (Secale) in sterilisierter Erde angesät und im Gewächshaus gezogen. Die Getreidesprösslinge werden 5 Tage nach Aussat mit einer Spritzbrühe des Wirkstoffes behandelt. Die Blattapplikation entsprach 6 kg Wirkstoff pro Hektar. Die Auswertung erfolgt nach 21 Tagen. Die Verbindungen 1, 2 und 3 hemmen in diesem Versuch den Wuchs der Getreidesprösslinge zwischen 8 und 19 % gemessen an unbehandelten Sprösslingen.

**Patentansprüche**

1. 2,4-Diamino-6-halogen-5-trifluormethylpyrimidin der Formel (I)

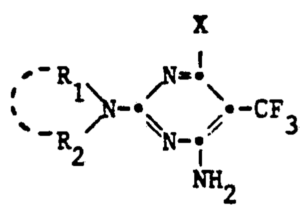

I

worin
X ein Halogenatom,
$R_1$ und $R_2$ unabhängig voneinander je Wasserstoff $C_1$-$C_6$ Alkyl, $C_3$-$C_6$-Cycloalkyl oder zusammen eine $C_3$-$C_7$ Alkylenbrücke bedeuten.

2. Die 2,4-Diamino-6-halogen-5-trifluormethylpyrimidine der Formel (I), Anspruch 1, in denen X Fluor oder Chlor bedeutet und $R_1$ und $R_2$ die im Anspruch 1 gegebene Bedeutung haben.

3. 2,4-Diamino-6-fluor-5-trifluormethylpyrimidine gemäss Anspruch 1, in denen $R_1$ und $R_2$ die im Anspruch 1 gegebene Bedeutung haben.

4. 2,4-Diamino-6-fluor-5-trifluormethylpyrimidin gemäss Anspruch 1.

5. 4-Amino-2-methylamino-6-fluor-5-trifluormethylpyrimidin gemäss Anspruch 1.

6. 4-Amino-2-dimethylamino-6-fluor-5-trifluormethylpyrimidin gemäss Anspruch 1.

7. Verfahren zur Herstellung der 2,4-Diamino-6-halogen-5-trifluormethylpyrimidin der Formel (I), Anspruch 1, dadurch gekennzeichnet, dass man ein 2,4,6-Trihalogen-5-trifluormethylpyrimidin der Formel (II)

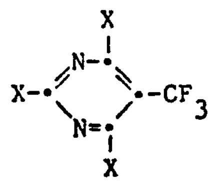

II

worin X die im Anspruch 1 gegebene Bedeutung hat, in einem inerten Lösungsmittel, bei einer Temperatur zwischen -10°C und +50°C mit 2-3 Mol Aequivalenten einer wässrigen Lösung eines Amins der Formel (III)

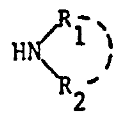

III

worin $R_1$ und $R_2$ die im Anspruch 1 gegebene Bedeutung haben, umsetzt, das entstandene 2-Amino-4,6-dihalogen-5-trifluormethylpyrimidin der Formel (IV) isoliert

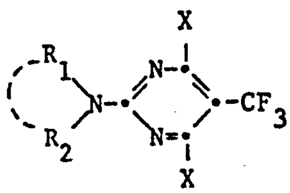

IV

worin $R_1$, $R_2$ und X die im Anspruch 1 gegebene Bedeutung haben, und es in einem inerten Lösungsmittel bei einer Temperatur zwischen +20 und +100°C mit 2-3 Mol wässriger Ammoniaklösung behandelt und das 2,4-Di-amino-6-halogen-5-trifluormethylpyrimidin der Formel (I) isoliert.

8. Herbizides und den Pflanzenwuchs regulierendes Mittel, dadurch gekennzeichnet, dass es als Wirkstoff eine Pyrimidin-Verbindung gemäss Anspruch 1 enthält neben Trägerstoffen und/oder andern inerten Zutaten.

9. Verfahren zur Regulierung des Pflansenwuchses in Nutzpflanzenkulturen, dadurch gekennzeichnet, dass man die Kulturfläche pre-emergent oder die Kultur post-emergent mit einen wirksamen Menge einer Pyrimidin-Verbindung gemäss Anspruch 1, behandelt.

## Claims

1. 2,4-Diamino-6-halo-5-trifluoromethylpyrimidine of the formula (I)

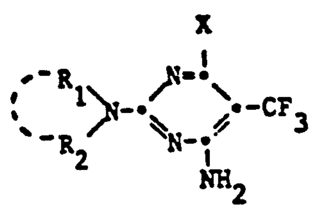

I

wherein

X is a halogen atom,

$R_1$ and $R_2$ independently of one another are each hydrogen, $C_1$-$C_6$-alkyl, $C_3$-$C_6$-cycloalkyl, or together form a $C_3$-$C_7$-alkylene bridge.

2. The 2,4-diamino-6-halo-5-trifluoromethylpyrimidines of the formula (I), claim 1, wherein X is fluorine or chlorine, and $R_1$ and $R_2$ have the meanings defined in claim 1.

3. 2,4-Diamino-6-fluoro-5-trifluoromethylpyrimidines according to claim 1, wherein $R_1$ and $R_2$ have the meanings defined in claim 1.

4. 2,4-Diamino-6-fluoro-5-trifluoromethylpyrimidine according to claim 1.

5. 4-Amino-2-methylamino-6-fluoro-5-trifluoromethylpyrimidine according to claim 1.

6. 4-Amino-2-dimethylamino-6-fluoro-5-trifluoromethylpyrimidine according to claim 1.

7. A process for producing the 2,4-diamino-6-halo-5-trifluoromethylpyrimidine of the formula (I), claim 1, characterised in that a 2,4,6-trihalo-5-trifluoromethylpyrimidine of the formula (II)

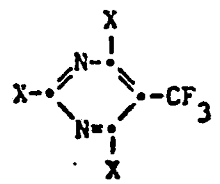

II,

wherein X has the meaning defined in claim 1, is reacted in an inert solvent and at a temperature of between -10°C and +50°C, with 2 - 3 mol equivalents of an aqueous solution of an amine of the formula (III)

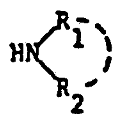

III,

wherein $R_1$ and $R_2$ have the meanings defined in claim 1, the resulting 2-amino-4,6-dihalo-5-trifluoromethylpyrimidine of the formula (IV)

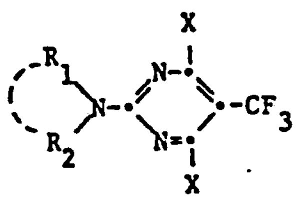

IV,

wherein $R_1$, $R_2$ and X have the meanings defined in claim 1, is isolated and treated in an inert solvent, at a temperature of between +20° and +100°C, with 2 - 3 mols of an aqueous ammonia solution, and the 2,4-diamino-6-halo-5-trifluoromethylpyrimidine of the formula (I) is isolated.

8. A herbicidal and plant-growth-regulating composition, characterised in that it contains as active ingredient a pyrimidine compound according to claim 1, together with carriers and/or other inert additives.

9. A process for regulating plant growth in crops of useful plants, characterised in that the cultivated area is subjected to pre-emergence treatment, or the crop is subjected to post-emergence treatment, with an effective amount of a pyrimidine compound according to claim 1.

**Revendications**

1. 2,4-diamino-6-halogène-5-trifluorométhylpyrimidine de formule (I)

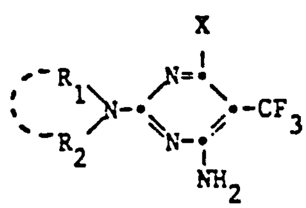

I

dans laquelle

X représente un atome d'halogène,

$R_1$ et $R_2$ représentent chacun indépendamment l'un de l'autre un hydrogène, un alcoyle en $C_1$ à $C_6$, un cycloalcoyle en $C_3$ à $C_6$ ou ensemble un pont alcoylène en $C_3$ à $C_7$.

2. Les 2,4-diamino-6-halogène-5-trifluorométhylpyrimidine de formule (I) de la revendication 1, dans lesquels X représente un fluor ou un chlore et $R_1$ et $R_2$ ont la signification donnée dans la revendication 1.

3. 2,4-diamino-6-fluoro-5-trifluorométhylpyrimidine selon la revendication 1, dans lesquels $R_1$ et $R_2$ ont la signification donnée dans la revendication 1.

4. 2,4-diamino-6-fluoro-5-trifluorométhylpyrimidine selon la revendication 1.

5. 4-amino-2-méthylamino-6-fluoro-5-trifluorométhylpyrimidine selon la revendication 1.

6. 4-amino-2-diméthylamino-6-fluoro-5-trifluorométhylpyrimidine selon la revendication 1.

7. Procédé de préparation de la 2,4-diamino-6-halogène-5-trifluorométhylpyrimidine de formule (I) de la revendication 1, caractérisé en ce qu'on fait réagir une 2,4,6-trihalogène-5-trifluorométhylpyrimidine de formule (II)

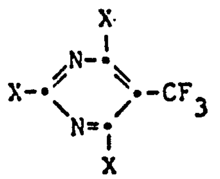

II

dans laquelle X a la signification donnée dans la revendication 1, dans un solvant inerte, à une température comprise entre -10°C et +50°C, avec de 2 à 3 équivalents molaires d'une solution aqueuse d'une amine de formule (III)

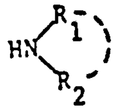

III

dans laquelle $R_1$ et $R_2$ ont la signification donnée dans la revendication 1, en ce qu'on isole la 2-amino-4,6-dihalogène-5-trifluorométhylpyrimidine apparue de formule (IV)

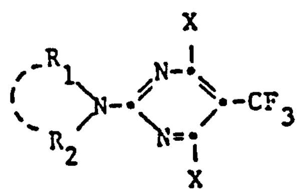

IV

dans laquelle $R_1$, $R_2$ et X ont la signification donnée dans la revendication 1, et on la traite dans un solvant inerte à une température comprise entre +20 et +100°C avec de 2 à 3 moles de solution aqueuse d'ammoniac et on isole la 2,4,-diamino-6-halogène-5-trifluorométhylpyrimidine de formule (I).

8. Agent herbicide et de régulation de la croissance des plantes, caractérisé en ce qu'il contient comme matière active un composé de pyrimidine selon la revendication 1 à côté de supports et/ou d'autres additifs inertes.

9. Procédé de régulation de la croissance végétale dans les cultures de plantes utiles, caractérisé en ce qu'on traite la surface cultivée en pré-levée ou la culture en post-levée avec une quantité efficace d'un composé de pyrimidine selon la revendication 1.